# EUROPEAN PATENT APPLICATION

(11) **EP 4 290 216 A1**
(43) Date of publication of application: **13.12.2023**
(21) Application number: 22305835.5
(22) Date of filing: 09.06.2022
(51) Int. Cl.: G01N 21/03, G01N 21/31, G01N 21/51, G01N 21/64

(54) **MULTI-ANALYSIS DEVICE AND METHOD**

(71) Applicant: Usense, 91300 Masy (FR)
(72) Inventor: LEMETAIS, Guillaume, 91300 Massy (FR); DESCHAMPS, Julie, 91300 Massy (FR)
(74) Representative: Icosa

(57) **Abstract**

The present invention relates to an analysis device (1) configured to simultaneously measure multiple parameters of a biological sample, said device comprising: at least one sample container (11) configured to contain a biological sample; and at least one measuring base (12) configured to cooperate with the sample container (11), wherein said measuring base (12) comprises: at least one electroanalytical sensor; a lighting module (126) configured to emit light in said biological sample through the sample container (11); and an optical sensor (127) configured to receive light emitted by said biological sample, light transmitted through said biological sample and/or light scattered in said biological sample; wherein the sample container (11) and the measuring base (12) comprise mutual cooperation means (111, 121). The present invention also relates to a method of analysis of a biological sample implemented by said analysis device (1) and the use of said analysis device (1).

## Description

### FIELD OF INVENTION

The present invention relates to the field of multi-analysis of a biological sample. In particular, the invention relates to a device and methods configured to simultaneously measure multiple parameters of a biological sample.

### BACKGROUND OF INVENTION

Analysis of biological samples often requires use of different machines that measure different parameters or biomarkers concentration. This has several disadvantages: logistic challenges, requires highly trained staff and expensive equipment, the most important disadvantage being the time to generate a result.

In particular, clinical urine tests, also known as urinalysis, are an examination of urine for certain biomarkers. Urinalysis is widely used for health check and/or diagnosis of diseases.

Currently, urinalysis is mostly performed in medical laboratories using different machines that measure different parameters or biomarkers concentration. The main disadvantage of urinalysis performed in laboratories is the time to generate a result. Indeed, urine samples first need to be prepared and separated into several tubes to be tested in parallel on different machines measuring different parameters (optical or electrical) most often by chemical dosage. The main drawback of chemical dosage is the sample deterioration. Then, the results of each machine must be aggregated in a report and returned to the practitioner. The results are generated a long time (4h to 48h) after the sampling, causing a delayed diagnosis and generating unwanted and unnecessary stress to the patient.

Thus, there is a need for a device performing analysis of a biological sample and allowing a fast, thorough and reliable diagnosis by simultaneously measuring multiple parameters in a biological sample. Combining several measuring techniques such as visible spectrometry, near infrared spectrometry, autofluorescence spectrometry and electroanalytical methods using one and only analysis reusable device overcomes the drawbacks mentioned hereabove. Furthermore, such a device should be able to measure said multiple parameters through a sample container so that the user does not have to prepare or manipulate any biological sample.

The Applicant has found that this need could be met with a analysis device comprising a sample container designed to contain a biological sample and stay closed throughout the measurement and a measuring base comprising an electroanalytical sensor, a lighting module configured to emit light in said biological sample through the sample container, and an optical sensor configured to receive light emitted by said biological sample, light transmitted through said biological sample, and/or light scattered in said biological sample.

### SUMMARY

The present invention also relates to an analysis device configured to simultaneously measure multiple parameters of a biological sample, said device comprising:
- at least one sample container configured to contain a biological sample; and
- at least one measuring base configured to cooperate with the sample container, wherein said measuring base comprises:
   a. at least one electroanalytical sensor;
   b. a lighting module configured to emit light in said biological sample through the sample container; and
   c. an optical sensor configured to receive light emitted by said biological sample, light transmitted through said biological sample and/or light scattered in said biological sample;
wherein the sample container and the measuring base comprise mutual cooperation means.

In one embodiment, the biological sample is selected in the group comprising blood, urine, lymph, fluidified feces, adipose tissue, bone marrow, cerebrospinal fluid, sperm, cord blood, milk, or saliva. In one embodiment, the at least one sample container comprises at least one electrically conductive contact configured to cooperate with the electroanalytical sensor of the measuring base so that current can flow between said electroanalytical sensor and the biological sample to measure at least one electroanalytical parameter of said sample. In one embodiment, the device further comprises at least one sterilization base configured to clean and/or decontaminate the at least one sample container after use. In one embodiment, the electroanalytical sensor is configured to perform conductimetry, coulometry, potentiometry, amperometry, or voltammetry on the biological sample, preferably said electroanalytical sensor is a conductivity probe. In one embodiment, the electroanalytical sensor is configured to measure conductivity, direct current and/or alternative current at multiple frequencies, said frequencies being included in a range from about 1 Hz to 1 MHz. In one embodiment, the lighting module comprises:
- a NIR-Vis light source and emits light over a range from 390 nm to 2500 nm, preferably from 390 nm to 1100 nm;
- a UV light source and emits UV light over a range from 190 nm to 400 nm, preferably from 270 nm to 400 nm; and/or
- an IR light source and emits IR light over a range from 800 nm to 10000 nm, preferably from 800 nm to 2600 nm.

In one embodiment, the optical sensor collects light over a range from 400 nm to 2500 nm, preferably from 400 nm to 1100 nm. In one embodiment, the optical sensor collects light over a range from 800 nm to 10000 nm, preferably from 800 nm to 2600 nm. In one embodiment, the measuring base further comprises a temperature sensor and a temperature-controlled sample holder configured to control sample temperature. In one embodiment, the measuring base further comprises an ultrasound sensor. In one embodiment, the sample container comprises optical filters.

The present invention also relates to a method of analysis of a biological sample implemented by the analysis device according to the invention, said method comprising the following steps:
i. collecting a biological sample in the sample container;
ii. engaging the sample container in the measuring base by connecting their mutual cooperation means; and
iii. measuring the following physical properties of said biological sample:
   - at least one electroanalytical parameter; and
   - NIR-Vis spectrum, IR spectrum and/or fluorescence spectrum.

In one embodiment, engaging the sample container in the measuring base further comprises rotating the sample container to an angle ranging from 10° to 170°, preferably from 20° to 90°, more preferably of 45°, so that a measuring indicator of the sample container faces a corresponding measuring indicator of the measuring base. In one embodiment, the method further comprises a cleaning and decontaminating step (iv) of at least one sample container.

### DEFINITIONS

In the present invention, the following terms have the following meanings:
**"Autofluorescence spectrometry"** refers to the measurement of light emitted by a tissue or a solution after excitation of said tissue or solution with light at specific wavelength, in particular ultra-violet light.
**"Electroanalytical methods",** such as for example coulometry, potentiometry, amperometry, conductimetry or voltammetry, refer to electrochemical techniques to characterize reversibility of electron transfer and impedance at an electrode-solution interface. For example, the term "conductimetry" may refer to the measurement of electrolytic conductivity of a solution.
**"IR"** refers to infrared range of wavelengths, from 780 nm to 10000 nm.
**"NIR"** refers to near infrared range of wavelengths, from 780 nm to 2500 nm, preferably from 780 nm to 1100 nm.
**"NIR-Vis"** refers to near infrared and visible range of wavelengths from 390 nm to 2500 nm, preferably from 390 nm to 1100 nm.
**"Near Infrared spectrometry"** refers to a quantitative measurement of light absorbance in near infrared range, *i.e.* of the ratio of the passed light with respect to the incident light in the near infrared range. This technique allows the detection of molecules absorbing low-energy radiation.
**"UV"** refers to ultraviolet light, from 190 nm to 400 nm, preferably from 270 nm to 400 nm.
**"Visible spectrometry"** refers to the characterization of the light absorption of a sample in visible range. When related to the extraction of quantitative information, one usually measures the intensity of light transmitted or reflected by the sample (I) and the intensity of a reference light (I₀) which can represent:
   - the intensity of light emitted by the source,
   - the intensity of light incident on the sample,
   - or the intensity of light transmitted or reflected by a reference sample,
   Some calculations are thus performed and the ratio I/I₀ (usually called transmittance) or the decimal logarithm of this ratio (usually called absorbance) are calculated.

### DETAILED DESCRIPTION

The following detailed description will be better understood when read in conjunction with the drawings. For the purpose of illustrating, the device is shown in the preferred embodiments. It should be understood, however that the present invention is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

### Analysis device

The invention relates to an analysis device configured to simultaneously measure multiple parameters of a biological sample, said device comprising:
- at least one sample container configured to contain a biological sample; and
- at least one measuring base configured to cooperate with the sample container, wherein said measuring base comprises:
   a. at least one electroanalytical sensor;
   b. a lighting module configured to emit light in said biological sample through the sample container; and
   c. an optical sensor configured to receive light emitted by said biological sample, light transmitted through said biological sample and/or light scattered in said biological sample;
wherein the sample container and the measuring base comprise mutual cooperation means.

The analysis device provides a non-invasive scan of biological samples based on four technologies: visible spectrometry, near infrared spectrometry (or infrared spectrometry), autofluorescence spectrometry and electroanalytical methods (preferably conductimetry). It allows a thorough physico-chemical characterization of a biological sample.

The optical sensor is configured to receive light transmitted through said biological sample such as visible and near infrared light, thus allowing the analysis device to perform visible spectrometry, near infrared spectrometry on a biological sample. Concerning visible spectrometry, it is possible to detect biomarkers such as, for example, minerals (*e.g.* Na, K, Ca, Mg, Cl, P), creatinine, urea, urine osmolality, urine specific gravity, uric acid, urine pH, ammonium, citrate, oxalate, albumin and micro-albuminuria, total proteins, bilirubin, urobilinogen, red blood cells, white blood cells, ketones, glucose or presence of bacteria or crystals. Concerning near infrared spectrometry, it is possible to detect biomarkers such as, for example, minerals (*e.g.* Na, K, Ca, Mg, Cl, P), creatinine, urea, urine osmolality, urine specific gravity, uric acid, urine pH, ammonium, citrate, oxalate, albumin, total proteins, bilirubin, urobilinogen, red blood cells, white blood cells, ketones, glucose or presence of bacteria or crystals. Urine osmolality and urine specific gravity are biomarkers of hydration, very useful to determine how well the kidneys are working. Creatinine is also a biomarker that indicates the proper functioning of kidneys.

The optical sensor is also configured to receive light emitted by said biological sample to anticipate autofluorescence of urine, thus allowing the analysis device to perform autofluorescence spectrometry on a biological sample. Fluorescence spectrometry allows the detection of biomarkers such as, for example, red blood cells, bacteria, heavy metals, NADH (hydrogenated nicotinamide adenine dinucleotide), NADPH (Nicotinamide adenine dinucleotide phosphate), FAD (flavin adenine dinucleotide), elastin, collagens, tryptophan, porphyrins, riboflavin, bilirubin, flavoproteins, pteridines compounds (neopterin, pterine, xanthopterin, isoxanthopterin) or other endogenous fluorophores.

The optical sensor is also configured to receive light scattered in said biological sample configured to detect light in a wide range of wavelengths.

Preferably, the optical sensor is a multispectral optical sensor.

Finally, the electroanalytical sensor allows the analysis device to perform electrochemical measurement, such as conductimetry, coulometry, potentiometry, amperometry, or voltammetry, on a biological sample. As an example, the conductivity of urine arises mainly from the mobility of the constituents (hydrated ions) present in the sample and therefore gives a measure of the ability of the sample to conduct a charge applied to it. Thus, by measuring the conductivity of a biological sample, it is possible to determine the concentration of ions (for example Na⁺, K⁺, Ca²⁺, Mg²⁺, H⁺/CO₃⁻ or Cl⁻). Other electroanalytical methods allow the analysis device to perform analysis of uric acid, oxalate, citrate, creatinine, bilirubin, amino acids such as tryptophane or tyrosine, BNP (brain natriuretic peptide), hormones such as Beta-HCG, cortisol steroid hormones, anti-mullerian hormone (AMH) or thyroid hormones, and marker of collagen degradation, in said sample.

Combining biomarkers detection by electroanalytical method and optical spectrometry is particularly advantageous as it allows for a thorough, and yet fast, scan of the biological sample, determining simultaneously the presence/absence and/or concentration of several biomarkers in a single biological sample. It also provides a better result, *i.e.* more precise, less false negative, than processing separately optical and electric measures. In addition, these measurements do not degrade the sample and can be repeated several times without impact on any future dosage. Finally, concomitant measurement of sample temperature allows to improve precision of optical and electroanalytical measurement.

For example, near-infrared spectrometry and electroanalytical measurement are complementary to determine precisely mineral concentration (*e*.*g*. Na, K, Ca, Mg, Cl, P). Indeed, inorganic ions in aqueous solutions do not directly absorb NIR light but influence spectral patterns at specific wavelength through ion-water interactions. Similarly, urine saturation and crystallization (*e*.*g*. presence of Calcium Oxalate crystals) can be detected optically. Thus, the optical spectrum brings both qualitative and quantitative information. This first estimation of each mineral concentration is completed by electroanalytical measurement, such as, for example, conductimetry measurement, that reflects total concentration of cations and anions in solution, each ion having a specific molar conductivity. The electroanalytical measures at different frequencies allowing the precise determination of each ion concentration. Moreover, as the mobility of ions increase with temperature, a simultaneous measurement of temperature on top of optical spectrum and electroanalytical measurement allows an even more precise measure of mineral concentration.

Moreover, visible, NIR and IR spectra contain specific wavelengths that are heavily associated with similar urine biomarkers. Thus, combination of these spectral information significantly improves biomarker concentration prediction. For example, information can be found on osmolality below 700 nm, between 800 nm and 850 nm, around 1000 nm, around 1150 nm and above 1200 nm, meaning all ranges contain information, sometimes redundant and sometimes not, allowing to improve osmolality measurement. Finally, fluorescence spectroscopy is used to identify specific biomarkers in combination with visible spectra. For example, hematuria can cause urine color change from light yellow to pink or red detectable in the visible spectrum. In that case, the presence of blood can be confirmed by fluorescence by measuring emission peak occurring at 450-520 nm.

To perform the biomarkers detection, the sample container is filled with a biological sample, engaged in a measuring base which is activated to perform the measurements, for example by a rotation of the sample container. Said sample container is then cleaned and/or disinfected after use.

The analysis device advantageously allows for the measurement of multiple parameters through the sample container. Indeed, throughout the measurement, the sample container stays closed so that the user does not have to prepare or manipulate any biological sample, avoiding any biological contamination from the biological sample to the environment and from the environment to the biological sample.

The biological sample can be provided by a human or an animal, *e.g.* cattle, sheep, pigs, horses or any other animal.

According to one embodiment, the biological sample is selected in the group comprising blood, urine, lymph, fluidified feces, adipose tissue, bone marrow, cerebrospinal fluid, sperm, cord blood, breast milk, tears, or saliva.

In a preferred configuration of this embodiment, the biological sample is urine so that the analysis device of the invention is a urinalysis device and the sample container is a urine container.

According to one embodiment, the sample container comprises male cooperation means, hereafter called engaging means, and the measuring base comprises female cooperation means, hereafter called receiving means. The engaging means of the sample container are configured to engage in the receiving means of the measuring base so that said sample container is removably connected to said measuring base. Preferably, a rotation movement is required to fully engage the sample container in the measuring base, triggering automatically the measurement after a few seconds. When engaged, the sample container cannot be unintentionally removed from the measuring base during measurement of biological sample parameters, thereby creating a stable coupling.

In a specific configuration, the male cooperation means is a screw thread and the female cooperation means is an internal thread.

In another specific configuration, the male cooperation means is a protrusion of specific shape, for example hexagonal, and the female cooperation means is a recess with a complementary shape to the protrusion.

According to one embodiment, the measuring base has a cylindrical shape, a hexagonal shape, or a rectangular shape. These shapes allow for a plurality of measuring bases to be connected together by at least one side, forming a matrix configuration.

According to one embodiment, the measuring base has a diameter ranging from 10 cm to 80 cm, preferably from 15 cm to 50 cm, more preferably from 20 cm to 40 cm.

Advantageously, the measuring base comprises a cover. Closing the cover after insertion of a sample container in the measuring base advantageously allows for the creation of a dark analysis chamber to avoid surrounding light pollution in the sample container.

According to one embodiment, the measuring base comprises an internal housing configured to accommodate a sample container and an external surface. Advantageously, the internal housing defines an empty space in which a sample container can be placed.

In a particular configuration of this embodiment, the internal housing has a diameter ranging from 3 cm to 60 cm, preferably from 4 cm to 30 cm, more preferably from 5 cm to 20 cm.

In a particular configuration of this embodiment, the internal housing comprises at least one wall and a base perpendicular to said wall, the at least one wall hosting the optical sensor, lighting module and electroanalytical sensor, i.e. the electrodes of the electroanalytical sensor. Preferably, the optical sensor and the lighting module are opposed to one another (*i.e.* facing each other).

In another particular configuration of this embodiment, the internal housing comprises at least two walls and a base perpendicular to said walls, the walls hosting the optical sensor, lighting module and electroanalytical sensor. Preferably, the walls hosting the optical sensor and the lighting module are opposed to one another (*i.e.* facing each other).

Preferably, the distance between the base of the internal housing and at least one sensor selected among: optical sensor, lighting module and electroanalytical sensor is less than 5 cm, more preferably less than 3 cm.

According to one embodiment, the optical sensor and the lighting module are opposed to one another (*i.e.* facing each other) and separated by a distance ranging from 1 mm to 30 mm, preferably from 1 mm to 15 mm. The biological sample contained in the sample container placed in the internal housing fills the empty space defined by said internal housing, between the optical sensor and the lighting module. The distance separating the optical sensor and the lighting module defines the optical path length.

In a particular configuration of this embodiment, the distance between the base of the internal housing and at least one sensor selected among: optical sensor, lighting module and electroanalytical sensor, is inferior to the distance between said at least one sensor and the top of the internal housing. Advantageously, said at least one sensor, preferably all sensors, are located close to the base of the internal housing so that measurement is performed on the inferior part of the sample container so that the quantity of biological sample needed for said measurement can be minimized.

According to one embodiment, the electrodes of the electroanalytical sensor are located next to each other. In an alternative configuration, electrodes of the electroanalytical sensor are facing each other.

Advantageously, the measuring base is not disposable. In the event that some biological sample is spilled on the measuring base, said base can be cleaned.

According to one embodiment, the electroanalytical sensor is configured to use direct current.

According to one embodiment, the electroanalytical sensor is configured to measure conductivity, direct current and/or alternative current (by conductimetry, voltammetry, potentiometry, coulometry and/or amperometry), at one or multiple frequencies, said frequencies being included in a range from about 1 Hz to 1 MHz, preferably from about 1 Hz to 100 kHz. In a preferred configuration of this embodiment, said frequencies are included in a range from about 10 Hz to 10 kHz. Having higher frequencies would lead to high power consumption and more complexity of the device. Preferably, the electroanalytical sensor is configured to measure conductivity of a sample.

According to one embodiment, the electroanalytical sensor comprises two or more electrodes.

In a preferred configuration, the electroanalytical sensor is a conductivity probe. In particular, said conductivity probe may be configured to measure direct current conductivity.

According to one embodiment, the lighting module comprises a NIR-Vis light source and emits light over a range from 390 nm to 2500 nm, preferably from 390 nm to 1100 nm. In a particular configuration of this embodiment, the NIR-Vis light source is a LED (light emitting diode), a laser, a superluminescent LED (sLED), or a filament lamp.

According to one embodiment, the lighting module comprises a UV light source and emits UV light over a range from 190 nm to 400 nm, preferably from 270 nm to 400 nm, more preferably from 365 nm to 400 nm. In a particular configuration of this embodiment, the UV light source is a UV LED (light emitting diode), a laser, a superluminescent LED (sLED), or a filament lamp.

According to one embodiment, the lighting module comprises an IR light source and emits IR light over a range from 780 nm to 10000 nm, preferably from 800 nm to 2600 nm.

According to one embodiment, the optical sensor collects light over a range from 400 nm to 2500 nm, preferably from 400 nm to 1100 nm.

According to one embodiment, the optical sensor collects light over a range from 800 nm to 10000 nm, preferably from 800 nm to 2600 nm.

According to one embodiment, the optical sensor comprises at least one light detector, preferably two or more light detectors. In a specific configuration of this embodiment, the optical sensor comprises a first light detector collecting light over a range from 400 nm to 2500 nm, preferably from 400 nm to 1100 nm, *i.e.* a visible light collector, and a second light detector collecting light over a range from 800 nm to 10000 nm, preferably from 800 nm to 2600 nm, *i.e.* an infrared light collector.

According to one embodiment, the optical sensor is a miniaturized spectrometer or an interferometer.

According to one embodiment, the measuring base further comprises a temperature sensor, preferably an infrared temperature sensor. Said temperature sensor is located as described hereabove for other sensors. The temperature sensor allows the measurement of the temperature of the biological sample which will then be converted into an electrical signal associated with said temperature.

In a specific configuration of this embodiment, the measuring base further comprises a temperature-controlled sample holder to control sample temperature in relation to the temperature sensor.

According to one embodiment, the analysis device comprising:
- at least one sample container configured to contain a biological sample; and
- at least one measuring base configured to cooperate with the sample container, wherein said measuring base comprises:
   a. at least one electroanalytical sensor, preferably a conductivity probe;
   b. a lighting module configured to emit light in said biological sample through the sample container;
      wherein the lighting module comprises a NIR-Vis light source emitting light over a range from 390 nm to 2500 nm (preferably from 390 nm to 1100 nm), a UV light source emitting UV light over a range from 190 nm to 400 nm (preferably from 270 nm to 400 nm), and an IR light source emitting IR light over a range from 800 nm to 10000 nm (preferably from 800 nm to 2600 nm);
   c. an optical sensor configured to receive light emitted by said biological sample, light transmitted through said biological sample, and/or light scattered in said biological sample;
      wherein the optical sensor comprises a first light detector collecting light over a range from 400 nm to 2500 nm (preferably from 400 nm to 1100 nm), and a second light detector collecting light over a range from 800 nm to 10000 nm (preferably from 800 nm to 2600 nm); and
   d. a temperature sensor;
wherein the sample container and the measuring base comprise mutual cooperation means.

According to one embodiment, the measuring base further comprises an ultrasound sensor and emitter. Said ultrasound sensor and emitter are located as described hereabove for other sensors. Said ultrasound sensor and emitter provide information on physical, mechanical and chemical properties of the biological sample such as density, presence of cells and/or crystals.

According to one embodiment, the measuring base further comprises a sensor configured to perform Raman spectroscopy measurement, Electrochemical Impedance Spectroscopy, or Fourier transform infrared spectroscopy measurement (FTIR) on the biological sample.

According to one embodiment, the measuring base further comprises a barcode reading system to identify the sample container. In this embodiment, the sample container comprises a barcode for identification purposes. Advantageously, this allows identity vigilance so that results are assigned to the right subject. Also, if many measurements are performed in parallel on distinct samples, this automatic identification of each sample and attribution of said identity to the results saves time for the user who does not have to manually label each sample, and prevent human errors on the identification of the samples.

According to one embodiment, the measuring base further comprises a scale to determine the weight of the biological sample contained in the sample container. Preferably, the scale is located on the base of the internal housing of the measuring base so that the sample container weights on it. Advantageously, knowing the weight of the biological sample leads to the volume of the biological sample and allows for the determination of fine concentrations.

According to one embodiment, the measuring base further comprises a rechargeable battery. In a specific configuration of this embodiment, the rechargeable battery is configured to work during 24h without charging. In order to preserve the battery, a stand-by mode can be automatically activated when the device is not in use. In another specific configuration of this embodiment, the rechargeable battery is configured to charge quickly, for example, the battery is fully charged after 1h. Preferably, the rechargeable battery is selected among lithium-ion battery, LiCFₓ battery, Li-FeS₂ battery, LiFePO₄ battery, Li-SO₂ battery, Li-I₂ battery, Li-Ag₂CrO₄ battery, Li-Ag₂V₄O₁₁ battery, Li-SVO battery, Li-CSVO battery, or lithium polymer battery. Preferably, the rechargeable battery is a lithium-based battery.

Alternatively, the measuring base is configured to charge via a wire connection.

According to one embodiment, the measuring base further comprises a connectivity system allowing for data transfer. In a specific configuration of this embodiment, the measuring base is configured to communicate via wireless connection with a computing module such as for example a smartphone, a tablet or a computer, via Bluetooth or wifi. In another specific configuration of this embodiment, the measuring base is configured to communicate via wired connection with a computing module such as for example a smartphone, a tablet or a computer, via an USB or ethernet cable.

According to one embodiment, the analysis device further comprises at least one display module configured to display data collected by the sensors/probe of the device and/or results obtained after data treatment. Said display module may be a screen located on the external surface of the measuring base, and is configured to display information such as subject identity, sample reference and/or measurement results.

According to one embodiment, the analysis device further comprises an activation button (also called ON/OFF button) located on the external surface of the measuring base.

According to one embodiment, the analysis device further comprises at least one sterilization base configured to clean and/or decontaminate the sample container after use. The sample container is placed facing downward in the sterilization base to be cleaned and/or decontaminated after cleaning with water and detergent.

According to one embodiment, the sterilization base comprises a cover to protect the user against UV for example in the case of a UV disinfection.

According to one embodiment, the sterilization base has dimensions and shapes as described hereabove for the measuring base.

In a specific configuration of this embodiment, the sterilization base can clean and/or decontaminate the sample container by UV exposition, ultrasounds, autoclave, or using a disinfection solution.

According to one embodiment, the sterilization base comprises a disinfection solution (also called cleaning solution).

In a particular configuration of this embodiment, the disinfection solution is an aqueous solution comprising surfactants. This is particularly advantageous as said solution forms a foam, preferably a thermoformed foam, which helps to prevent the attachment of biological molecules on the sample container.

In a particular configuration of this embodiment, the disinfection solution comprises didecyldimethylammonium chloride, polyhexamethylene biguanide hydrochloride, detergent complexes (polyalkoxylated fatty alcohol, lauryldimethylamine oxide), sequestering and dispersing agent.

In an alternative configuration of this embodiment, the disinfection solution refers to an alternance of water and solvent (*e.g.* ethanol), *i.e.* the sample container is first plunged in water then in solvent, or first rinsed in water then solvent.

According to one embodiment, the analysis device comprises an assembly of a plurality of measuring bases configured to each receive a sample container. Advantageously, this allows for the simultaneous analysis of a plurality of biological samples.

According to one embodiment, the analysis device comprises an assembly of a plurality of measuring bases and at least one sterilization base. Advantageously, this allows for the simultaneous analysis of a plurality of biological samples and cleaning of the sample containers directly after use.

In a specific configuration, the analysis device comprises an assembly base configured to host said assembly of a plurality of measuring bases. Preferably, said assembly base can host 1 to 10 bases (measuring and/or sterilization bases), more preferably 2 to 5 bases (measuring and/or sterilization bases).

Said bases (measuring and/or sterilization bases) may also assemble independently from an assembly base.

In order to assemble together, bases (measuring and/or sterilization bases) may comprise assembling means such as, for example magnets or hooks.

According to one embodiment, the sample container comprises an analysis chamber located at the bottom of the sample container and configured to contain biological sample in a small volume for measurement purposes. Preferably the analysis chamber has a height ranging from 0.5 mm to 5 mm, a width ranging from 0.5 mm to 10 mm.

According to one embodiment, the sample container comprises at least one electrically conductive contact, preferably a plurality of electrically conductive contacts, configured to cooperate with the electroanalytical sensor of the measuring base so that current can flow between the electroanalytical sensor of the measuring base and the biological sample to measure the conductivity, voltammetry, amperometry, coulometry and/or potentiometry, of said sample. Advantageously, the conductivity, voltammetry, amperometry, coulometry and/or potentiometry, of the biological sample can be measured without opening the sample container.

In a specific configuration, the electrically conductive contact comprises one or a plurality of small studs or protrusion.

According to one embodiment, the sample container is a tube or a biological sample collection container.

According to one embodiment, the sample container has a diameter ranging from 1 cm to 10 cm, preferably from 2 cm to 5 cm. This diameter defines the volume of sample that light will pass through.

In a specific configuration of this embodiment, the sample container is configured to contain from 1 ml to 100 ml of biological sample, preferably from 5 ml to 50 ml, more preferably from 5 ml to 10 ml.

According to one embodiment, the sample container is not disposable. Said sample container is configured to be cleaned after use. In particular, said sample container can be cleaned by placing it in a sterilization base as described hereabove.

Alternatively, the sample container is disposable.

According to one embodiment, the sample container comprises a material allowing passage of light, i.e. optically transparent to UV light, visible light, NIR light and/or IR light, shock resistant and scratch resistant, such as for example quartz or glass.

According to one embodiment, the sample container comprises a material resistant to cleaning and disinfection.

According to one embodiment, the sample container comprises optical filters. Advantageously, optical filters allow:
- the measurement of absorbance and/or fluorescence at specific wavelengths;
- focusing of the light with optical lenses;
- polarization of the light to detect specific markers such as the presence of crystals.

According to one embodiment, the device further comprises a robotic arm configured to manipulate the sample container so that it can be placed in a measuring base and/or in a sterilization base without the need for the user to touch it.

### Method of sample analysis

The present invention also relates to a method of analysis of a biological sample implemented by the analysis device according to the invention, said method comprising the following steps:
i. collecting a biological sample in the sample container;
ii. engaging the sample container in the measuring base by connecting their mutual cooperation means; and
iii. measuring the following physical properties of said biological sample:
   - at least one electroanalytical parameter; and
   - NIR-Vis spectrum, IR spectrum and/or fluorescence spectrum.

The analysis device, sample container, measuring base are as described hereabove.

According to one embodiment, the biological sample is selected in the group comprising blood, urine, lymph, fluidified feces, adipose tissue, bone marrow, cerebrospinal fluid, sperm, cord blood, milk, or saliva.

In a preferred configuration of this embodiment, the biological sample is urine so that the device of the invention is a urinalysis device, the sample container is a urine container and the method of the invention is a method of urinalysis.

The electroanalytical parameter refers to conductivity, voltammetry, amperometry, coulometry and/or potentiometry.

Prior to step ii), the analysis device may be switched on. In a specific configuration, said device further comprises a first external LED located on the measuring base, preferably on its external surface, such as for example a blue LED, green LED or red LED, preferably a blue LED that indicates if said device is well switched on by emitting light.

Prior to step ii) or step iii), the analysis device may also be connected to a computing module, such as a computer, a smartphone or a tablet, via a wireless connection or wired connection, or to an assembly base as described hereabove.

To measure the physical properties of the biological sample, the analysis device is activated by engaging the sample container in the measuring base. Then, the analysis device performs the measurements of physical properties of said biological sample. The start of the measurement may be ordered manually by the user or automatically when the device detects that the sample container is correctly engaged in the measuring base.

In a specific configuration, said analysis device further comprises a second external LED located on the measuring base, preferably on its external surface, such as for example a blue LED, green LED or red LED, preferably a green LED that indicates if measurement was performed by emitting light and/or vibrating.

The method of the invention does not require that the sample is prepared, separated, or subjected to any treatment before engaging the sample container in the measuring base. Indeed, said sample container can be engaged in the measuring base immediately after collection of the biological sample. Nonetheless, the biological sample may be mixed with a reagent prior immersion of the analysis device. Such a reagent may be a fluorescent probe to highlight the presence of molecules or cells of interest such as bacteria, leukocytes, glucose, sodium, calcium, chlorine, hormones (beta-HCG, FMH, steroids), DNA or RNA, or a signal amplifier such as gold nanoparticles to amplify the signal.

According to one embodiment, the measurement of physical properties of the biological sample can start directly and automatically after placing the sample container in a measuring base.

According to an alternative embodiment, engaging the sample container in the measuring base further comprises rotating the sample container to an angle ranging from 10° to 170°, preferably from 20° to 90°, more preferably of 45°, so that a measuring indicator of the sample container faces a corresponding measuring indicator of the measuring base. This rotation ensures that all sensors are well placed to perform measurement, in particular that the electroanalytical sensor is connected with the contact of the sample container.

In a specific configuration of this embodiment, the measuring indicator of the sample container is an arrow, for a low-tech visual, and the measuring indicator of the measuring base is a LED, for a high-tech signal. The LED of the lighting module can also play the role of measuring indicator of the base.

According to one embodiment, the method further comprises a cleaning and decontaminating step (iv) of the sample container, in particular by placing the sample container in a sterilization base as described hereabove. Advantageously, the cleaning step (iv) is a fast, non-dangerous step that allows the user to reuse the sample container almost immediately.

In a specific configuration of this embodiment, step (iv) comprises the cleaning by rinsing the sample container with water (outside of the bases, for example at a water point such as a sink) and/or decontamination in the sterilization base (also called herein disinfection) by UV exposition, autoclave, or using a disinfection solution.

According to one embodiment, step (iii) further comprises measuring at least one physical parameter of the biological sample by Raman spectroscopy measurement, Electrochemical Impedance Spectroscopy, or Fourier transform infrared spectroscopy measurement (FTIR).

### Use of the analysis device

This invention also relates to a use of the analysis device for analysis of a biological sample as described hereabove.

This invention also relates to a use of the analysis device for urine analysis. Said urine can be human urine or animal urine.

While various embodiments have been described and illustrated, the detailed description is not to be construed as being limited hereto. Various modifications can be made to the embodiments by those skilled in the art without departing from the true spirit and scope of the disclosure as defined by the claims.

All the embodiments hereabove can be combined.

### BRIEF DESCRIPTION OF THE DRAWINGS

**Figure 1A** is a schematic view of the analysis device according to one embodiment.
**Figure 1B** is a schematic view of the analysis device according to another embodiment.
**Figure 1C** is a schematic view of the analysis device according to another embodiment.
**Figure 2A** is a schematic view of a sample container according to one embodiment.
**Figure 2B** is a schematic view of a sample container according to another embodiment.
**Figure 3** is a schematic view of the measuring base according to one embodiment.
**Figure 4A** is a schematic view of the sterilization base according to one embodiment.
**Figure 4B** is a schematic view of the sterilization base according to another embodiment.
**Figure 5A** is a schematic view of an assembly of measuring bases and a sterilization base.
**Figure 5B** is a schematic view of an assembly base.

### ILLUSTRATIVE EMBODIMENTS OF THE INVENTION

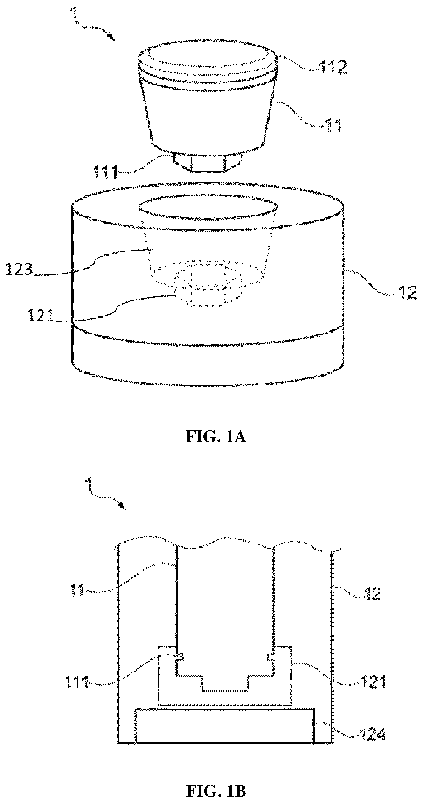

According to one embodiment illustrated in **Figure 1A****,** the analysis device 1 comprises:
- a sample container 11 configured to contain a biological sample, comprising a cap 112; and
- a measuring base 12 configured to cooperate with the sample container 11.

The sample container 11 and the measuring base 12 comprise mutual cooperation means (111, 121). In particular, the sample container 11 comprises male cooperation means 111 being a protrusion of specific shape (hexagonal represented in Fig. 1A) that engages with female cooperation means 121 of the measuring base 12, said female cooperation means 121 being a recess with a complementary shape to the protrusion.

According to one embodiment illustrated in **Figure 1B****,** the analysis device 1 comprises:
- a sample container 11 configured to contain a biological sample; and
- a measuring base 12 configured to cooperate with the sample container 11, comprising a sensing zone 124 wherein the sensors are located (optical module, optical sensor, electroanalytical sensor).

The sample container 11 and the measuring base 12 comprise mutual cooperation means (111, 121). In particular, the sample container 11 comprises male cooperation means 111 being a screw thread that engages with female cooperation means 121 of the measuring base 12, said female cooperation means 121 being an internal thread.

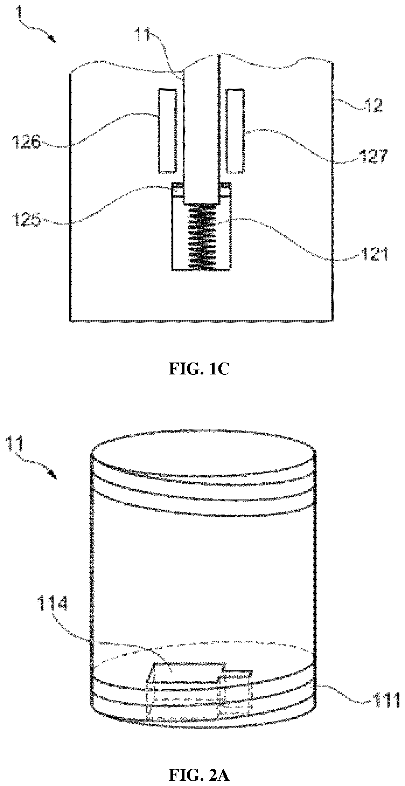

According to one embodiment illustrated in **Figure 1C****,** the analysis device 1 comprises:
- a sample container 11 configured to contain a biological sample, said sample container being a sampling tube; and
- a measuring base 12 configured to cooperate with the sample container 11, comprising:
   o an electroanalytical sensor 125, preferably a conductivity probe;
   o a lighting module 126 configured to emit light in said biological sample through the sample container 11; and
   o an optical sensor 127 configured to receive light emitted by said biological sample and/or light transmitted through said biological sample.

The sample container 11 and the measuring base 12 comprise mutual cooperation means (111, 121).

According to one embodiment illustrated in **Figure 2A****,** the sample container 11 is a urine collection container having a cylindrical shape and comprising:
- a screw thread configured to cooperate with a cap;
- an analysis chamber 114 at the bottom; and
- male cooperation means 111 being a screw thread configured to cooperate with female cooperation means of the measuring base (not illustrated on Fig. 2A).

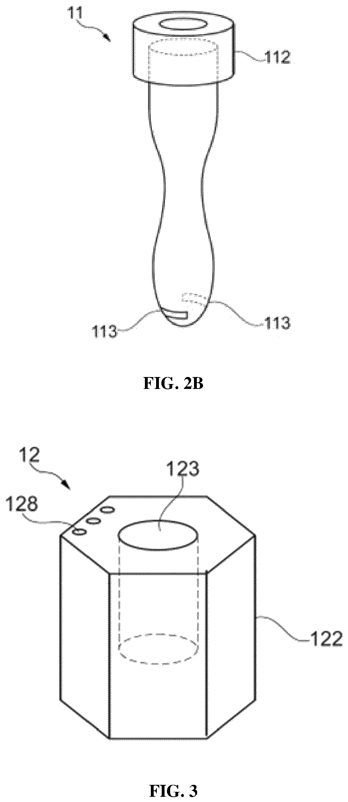

According to one embodiment illustrated in **Figure 2B****,** the sample container 11 is a sampling tube comprising:
- a cap 12, in particular a septum;
- electrically conductive contacts 113 configured to cooperate with the electroanalytical sensor of the measuring base (not illustrated on Fig. 2A).

According to one embodiment illustrated in **Figure 3****,** the measuring base 12 comprises:
- an internal housing 123 configured to accommodate a sample container;
- an external surface 122; and
- measuring indicators 128, preferably LEDs, showing the engagement of the sample container 11 in the measuring base 12 and the progress of the analysis.

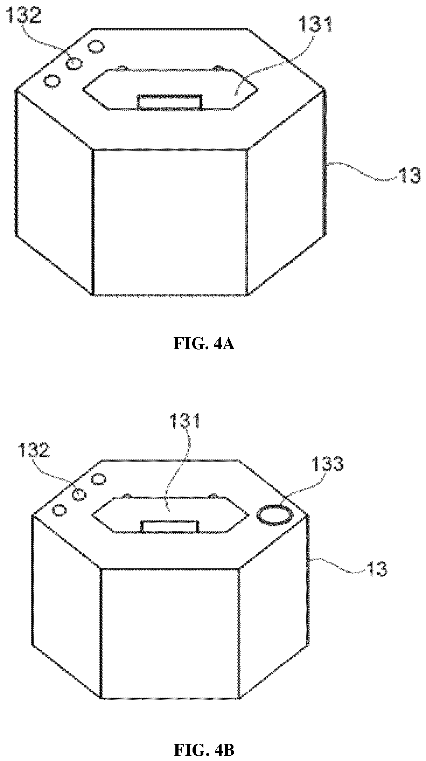

According to one embodiment illustrated in **Figure 4A****,** the sterilization base 13 comprises a cover 131 configured to protect the user from UV light used to disinfect the sample container 11 in the sterilization base 13, and indicators 132, preferably LEDs, showing presence of a sample container 11 in the sterilization base 13 and progress of the disinfection process.

According to one embodiment illustrated in **Figure 4B****,** the sterilization base 13 comprises:
- a cover 131 configured to protect the user from UV light used to disinfect the sample container 11 in the sterilization base 13;
- indicators 132, preferably LEDs, showing presence of a sample container 11 in the sterilization base 13 and progress of the disinfection process; and
- pumping means 133.

The sterilization base 13 of this embodiment is usable with sample tubes.

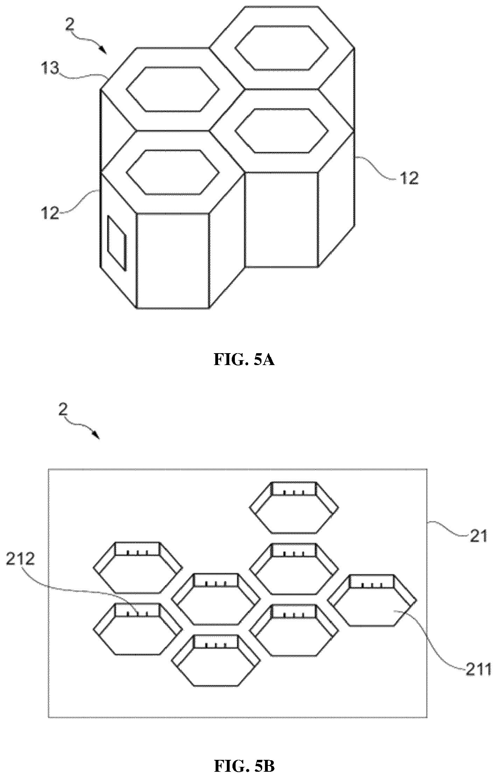

According to one embodiment illustrated in **Figure 5A****,** measuring bases 12 and sterilization base 13 can assemble together, by means of magnets or hooks, to form an assembly 2.

According to one embodiment illustrated in **Figure 5B****,** measuring bases 12 and sterilization base 13 can assemble together to form an assembly 2 on an assembly base 21. Said assembly base 21 comprises sites 211 having a shape complementary to the shape of the measuring base 12 and sterilization base 13, each site 211 comprising power supply contacts 212 to charge said bases (12, 13).

### EXAMPLES

The present invention is further illustrated by the following examples.

### Example 1:

### Methods

Freshly collected urine samples were collected in 10 mL sterile urine containers without additives/preservatives. Each urine container was placed in a measuring base and engaged by a rotation of 45°. Measuring bases were switched on and analytical measurements were performed.

To evaluate the analytical performances of the analysis device of the invention, reference testing was performed by a central Lab with gold-standard methodologies.

The same samples were analyzed in parallel by using the analysis device of the invention to measure optical spectrum (visible spectrometry, near infrared spectrometry, autofluorescence spectrometry) and electrical conductivity. Numerical data were then processed by specific algorithms to determine concentrations values for each sample: on one side concentrations values based on optical data, on the other side concentration values based on a combination of optical and electrical data.

The concentrations determined by the reference equipment were then used as control value and compared to both concentration values obtained by the analysis device: based on optical data and based on combination of optical and electrical data.

### Results

Table 1 shows correlations between biomarker concentration with gold-standard devices and results obtained by the analysis device with optical data, or with a combination of optical and electrical data.

**Table 1: Correlation between Gold Standard methodologies and the analysis device with or without electrical data**

| | Gold Standard vs analysis device with optical data | Gold Standard vs analysis device with combined optical and electrical data |
|---|---|---|
| Phosphorus | 0.65 | 0.85 |

We observed that compared to references, the values we obtain are more precise when algorithms are used on a combination of optical and electrical data instead of only optical data.

### Example 2:

### Materials:

Freshly collected early morning urine samples were collected. A selection was made on urolithiasis patients in order to evaluate crystal presence in said urine samples. Each primary urine sample was collected in 10 mL sterile urine containers without additives/preservatives.

Each urine container was placed in a measuring base and engaged by a rotation of 45°. Measuring bases were switched on and analytical measurements were performed.

Urine samples are analyzed by the analysis device to characterize physico-chemical profile obtained by optical analysis (visible spectrometry, near infrared spectrometry, autofluorescence spectrometry) and electrical analysis (conductimetry) in order to determine sample concentrations.

### Results

Urines profiles measured with the analysis device are highly specifics and vary from one individual to another. Theses variations depend on parameters such as individual state of health and may be induced by pathologies such as urolithiasis which cause crystals formation in the urine.

When analyzing samples, the data obtained by the instrument varies according to the physico-chemical parameters. For example, the conductivity varies according to ionic concentration and the presence of crystals in the samples. In healthy people, conductivity varies from 11.49 to 16.85 mS.cm⁻¹.

The measured conductivity value performed by the analysis device allowed to identify high crystal risk samples (conductivity > 25 mS.cm⁻¹) from healthy samples and to adapt algorithms predicting biomarker concentration. Crystal presence was confirmed by observing urine samples with contrast microscope equipped with a polarized light device.

### REFERENCES

1 - analysis device
11 - sample container
111 - cooperation means
112 - cap
113 - electrically conductive contact
114 - analysis chamber
12 - measuring base
121 - cooperation means
122 - external surface
123 - internal housing
124 - sensing zone
125 - electroanalytical sensor
126 - lighting module
127 - optical sensor
128 - measuring indicator
13 - sterilization base
131 - cover
132 - indicator
133 - pumping means
2 - assembly of bases
21 - assembly base
211 - site
212 - power supply contact

## Claims

1. An analysis device (1) configured to simultaneously measure multiple parameters of a biological sample, said device comprising:
- at least one sample container (11) configured to contain a biological sample; and
- at least one measuring base (12) configured to cooperate with the sample container (11), wherein said measuring base (12) comprises:
a. at least one electroanalytical sensor (125);
b. a lighting module (126) configured to emit light in said biological sample through the sample container (11); and
c. an optical sensor (127) configured to receive light emitted by said biological sample, light transmitted through said biological sample and/or light scattered in said biological sample;
wherein the sample container (11) and the measuring base (12) comprise mutual cooperation means (111, 121).

2. The analysis device (1) according to claim **1**, wherein the biological sample is selected in the group comprising blood, urine, lymph, fluidified feces, adipose tissue, bone marrow, cerebrospinal fluid, sperm, cord blood, milk, or saliva.

3. The analysis device (1) according to claim **1** or **2**, wherein the at least one sample container (11) comprises at least one electrically conductive contact (113) configured to cooperate with the electroanalytical sensor (125) so that current can flow between said electroanalytical sensor (125) and the biological sample to measure at least one electroanalytical parameter of said sample.

4. The analysis device (1) according to any one of claims **1** to **3**, further comprising at least one sterilization base (13) configured to clean and/or decontaminate the at least one sample container (11) after use.

5. The analysis device (1) according to any one of claims **1** to **3**, wherein the electroanalytical sensor (125) is configured to perform conductimetry, coulometry, potentiometry, amperometry, or voltammetry on the biological sample, preferably said electroanalytical sensor (125) is a conductivity probe.

6. The analysis device (1) according to claim **5**, wherein the electroanalytical sensor (125) is configured to measure conductivity, direct current and/or alternative current at multiple frequencies, said frequencies being included in a range from about 1 Hz to 1 MHz.

7. The analysis device (1) according to any one of claims **1** to **6**, wherein the lighting module (126) comprises:
- a NIR-Vis light source and emits light over a range from 390 nm to 2500 nm, preferably from 390 nm to 1100 nm;
- a UV light source and emits UV light over a range from 190 nm to 400 nm, preferably from 270 nm to 400 nm; and/or
- an IR light source and emits IR light over a range from 800 nm to 10000 nm, preferably from 800 nm to 2600 nm.

8. The analysis device (1) according to any one of claims **1** to **7**, wherein the optical sensor (127) collects light over a range from 400 nm to 2500 nm, preferably from 400 nm to 1100 nm.

9. The analysis device (1) according to any one of claims **1** to **8**, wherein the optical sensor (127) collects light over a range from 800 nm to 10000 nm, preferably from 800 nm to 2600 nm.

10. The analysis device (1) according to any one of claims **1** to **9**, wherein the measuring base (12) further comprises a temperature sensor and a temperature-controlled sample holder configured to control sample temperature.

11. The analysis device (1) according to any one of claims **1** to **10**, wherein the measuring base (12) further comprises an ultrasound sensor.

12. The analysis device (1) according to any one of claims **1** to **11**, wherein the sample container (11) comprises optical filters.

13. A method of analysis of a biological sample implemented by the analysis device (1) according to any one of claims **1** to **12**, said method comprising the following steps:
i. collecting a biological sample in the sample container (11);
ii. engaging the sample container (11) in the measuring base (12) by connecting their mutual cooperation means (111, 121); and
iii. measuring the following physical properties of said biological sample:
• at least one electroanalytical parameter; and
• NIR-Vis spectrum, IR spectrum and/or fluorescence spectrum.

14. The method according to claim **13**, wherein engaging the sample container (11) in the measuring base (12) further comprises rotating the sample container (11) to an angle ranging from 10° to 170°, preferably from 20° to 90°, more preferably of 45°, so that a measuring indicator of the sample container (11) faces a corresponding measuring indicator of the measuring base (12).

15. The method according to claim **13** or **14**, further comprising a cleaning and decontaminating step (iv) of at least one sample container (11).
